# EUROPEAN PATENT APPLICATION

(11) **EP 4 599 781 A1**
(43) Date of publication of application: **13.08.2025**
(21) Application number: 22961319.5
(22) Date of filing: 04.10.2022
(51) Int. Cl.: A61B 17/80, A61D 1/00

(54) **CLOSING WEDGE OSTEOTOMY PLATE, IN THE PROXIMAL PART OF THE ULNA IN PET ANIMALS**

(71) Applicant: Biosurgex, S.L., Las Palmas (ES)
(72) Inventor: BELMONTE AFONSO, Gonzalo, 35119 Pozo Izquierdo, Santa Lucía de Tirajana LAS PALMAS (ES); MONOPOLI ROCA, Angelo, 35119 Pozo Izquierdo, Santa Lucía de Tirajana LAS PALMAS (ES)
(74) Representative: Urizar Anasagasti, Jesus Maria
(86) International application number: PCT/ES2022/070630
(87) International publication number: WO 2024/074735

(57) **Abstract**

The present invention relates to a plate for proximal ulnar closing wedge osteotomy for pet animals, which when secured on top of the bone forms a connecting bridge between the two fragments into which the osteotomy (O) has divided the ulna (C), each of which is secured to the said plate by means of three screws, one of which is a compression screw (5) that is mounted in a hole (14) with an elongate slot that forms an inner ramp (141) running inwards from the distal to the proximal end of the plate (1), such that when the screw (5) is tightened, a compression effect is generated between the bone fragments of the distal and proximal part of the ulna, tending to close the wedge of the osteotomy (O); while the other securing screws (2, 3, 4, 6, 7) are of the polyaxial type in order to allow a certain range of angulation with respect to the plate (1) when secured.

## Description

### Technical field

The field of the invention is that of surgical instruments and devices; more specifically of cortical plates, that is, bone plates; and also of instruments and devices for holding or positioning cortical plates, or for compressing bones attached to cortical plates with means for distracting or compressing the bone or bones, the extension or compression force being caused by interaction of the plate hole and the screws.

The plate of the invention has been developed for use in a surgical technique called Proximal Ulnar Closing Wedge Osteotomy (PU-CWO).

Pet animals, especially medium and large dog breeds, often suffer from elbow pain due to joint incongruence, which causes a common condition leading to erosion of the joint cartilage. Joint incongruencies may be due to a misalignment between the ulnar and radial notch, causing a "step", or to a conformational incongruity.

The geometry of the lunate notch of the ulna should be semicircular for the joint to be congruent; however, when a conformational incongruity is present, the notch may be elliptical. This conformational incongruity during joint movement induces an increase in joint pressure, eventually damaging the cartilage covering the lunate bone of the ulnar and the radius, especially the distal medial part. Over time the joint develops osteoarthritis and pain.

This type of joint incongruity can be modified with an osteotomy in the caudal part of the ulnar to increase the radius of curvature of the lunate notch to relieve joint pressure and prevent the progression of osteoarthritis in the elbow. The plate of the invention facilitates the practice of a proximal ulnar closing wedge osteotomy (PU-CWO) in cases where the pet's elbow has a conformational incongruity.

### State of the art

A study of forelimb deformities in dogs described that 75% of these were due to ulnar anomalies which can be attributed to a multitude of aetiologies, with trauma being the most common cause of early closure of the distal growth plates. Many surgical techniques and preoperative planning methods have been described to correct these deformities, including surgeries involving osteotomy with either an opening or closing wedge technique (Ramadan et al. 1978, Morgan and Miller 1994 and Henney and Gambardella 1989). To date, this type of surgery has been performed on the distal area of the ulna, but never on the proximal area, where it is necessary to tackle the problems mentioned above and in the absence of specially designed plates for this area, there are few known surgical operations.

EP3738534A1 and other documents disclose osteosynthesis devices that comprise a bone plate with at least one through hole through which passes a screw the head of which can pivot at least about an axis, such that, when the plate is placed in the desired position on the bone, it is possible to orient the screw in the desired direction, which does not necessarily have to be orthogonal to the plate.

US3528085A discloses a bone compression plate having a length that can span adjacent sections of bone that have openings between them. At least one of the openings receives a screw having a head adjacent to one end with a lower surface that tapers inwardly towards the other end of the screw and having an inclined surface on the plate, which is positioned adjacent to the elongated opening between the bone sections, into which the inwardly tapering lower surface of the screw fits such that when the screw and the section of bone connected to the screw are moved, the movement is relative to the opening between the bone sections.

Document FR1505513A relates to an osteosynthesis plate in which at least one of its aligned holes comprises a longitudinal slot allowing a slight longitudinal displacement of the screw intended to pass through the slot, its lateral edges being inclined downwards, from the outside towards the inside, starting from its upper face towards its lower face, such that it can cooperate with the lower face of the head of a screw, same having a greater inclination, this lower face of the screw head being for example conical or hemispherical, thereby causing the insertion of the screw, at the end of its travel, its longitudinal displacement inside the groove in which it is fitted from the outside towards the inside.

Existing plates do not permit closing wedge osteotomy when performed on the proximal ulna due to interference with the medial humeral epicondyle at the joint and/or with the ulnar coronoid process.

### Explanation of the invention

Based on the prior art, one objective of the present invention is to provide a plate to modify the joint incongruence in the elbow (at the humerus-ulna contact surface) of some pet animals, by means of a proximal ulnar closing wedge osteotomy, to increase the radius of curvature of the lunate notch to relieve joint pressure and prevent the progression of osteoarthritis in the elbow.

In order to achieve the proposed objectives, which are mentioned in the previous paragraph, the invention proposes a plate, having the features of claim 1.

The function of this plate is to stabilise the ulnar fragments after application of the closing wedge osteotomy until osseointegration of the fragments is complete. It is worth mentioning that it is a plate that is mostly exposed to a high shear force that is located at the "neck" of the plate. This force is the result of the action of the triceps on the proximal ulna fragment, so it is sometimes necessary to complement the surgery with an additional normal plate on the caudal part of the ulna.

Although the positioning of the plate on the ulna is limited by its possible interference with the medial humeral epicondyle at the joint, with the ulnar coronoid process and the location chosen for the osteotomy, the plate of the invention allows a proximal ulnar closing wedge osteotomy to be performed in the majority of cases that the veterinarian may be faced with when correcting the joint incongruence that occasionally occurs in pet animals, especially medium and large dog breeds.

To achieve the above-mentioned objective, the plate has polyaxial securing screws, which allow a certain range of angulation of the proximal and distal screws, such that the veterinarian has a greater margin of placement of the plate to meet the needs in each context.

The distribution of the screws and the size of the plate are based on anatomical studies. In order to be able to adapt this plate to different dog breeds and sizes, in principle, 3 plates of different sizes have been developed.

Each ulna fragment is provided with at least 3 screws to completely limit the degrees of freedom of said fragments. The holes are arranged so as to be on top of the bone after osteotomies of up to 15°, although the common range of osteotomies required is between 8.5 and 11°.

The plate has a compression system to ensure that both bone fragments are in contact and integrate correctly. This system consists of a hole with a ramp which, when a compression screw with a spherical head is screwed in, induces a compression between the two bone fragments. Both the direction and the inclination of the ramp have been studied to prevent defects in the lunate notch of the ulna after compression is generated, taking into account that the osteotomy should not separate the ulna into two single fragments, but should be performed in such a way that the semilunate notch of the ulna remains intact, this being the connecting bridge between the fragments. It is important to keep the notch intact to prevent the incorporation of a possible "step" after compression of the fragments that could affect joint movement.

After applying the osteotomy, the fragments are joined together with a compression hand tool; however, once the plate is positioned, the fragments will be compressed under the effect of the compression screw. The direction of the ramp of the compression hole is not perpendicular to the median plane of the osteotomy as this could damage the bridge of the lunate notch of the ulnar or induce a fold in it. It is for this reason that the inclination with respect to the plate axis for the bridge to act as a hinge during compression is between 40-70°, the chosen inclination being 55°.

### Description of the drawings

As a complement to the present description, and for the purpose of helping to make the features of the invention more readily understandable, said description is accompanied by a set of drawings which, by way of illustration and not limitation, represent the following:
- Figure 1 shows a plate (1) secured in position over a closing wedge osteotomy (O) located on the proximal ulna (C) of a companion pet.
- Figure 2 shows a plan view of said plate (1).
- Figure 3 depicts respective plan and cross-sectional views of a plate (1) through the hole (14) in which the compression screw (5) is placed.
- Figures 4 and 5 represent respective views of the compression screw (5) in its starting position, before starting the closing action by compression of the wedge osteotomy (O) and after closing the same.

### Embodiment of the invention

The closing wedge osteotomy plate of the present invention is used in surgery to increase the radius of curvature of the semilunate notch (ES) of the ulna (C) by performing a proximal ulnar wedge osteotomy (O), opening outwards but keeping an area in the semilunate notch (ES) of the ulnar intact.

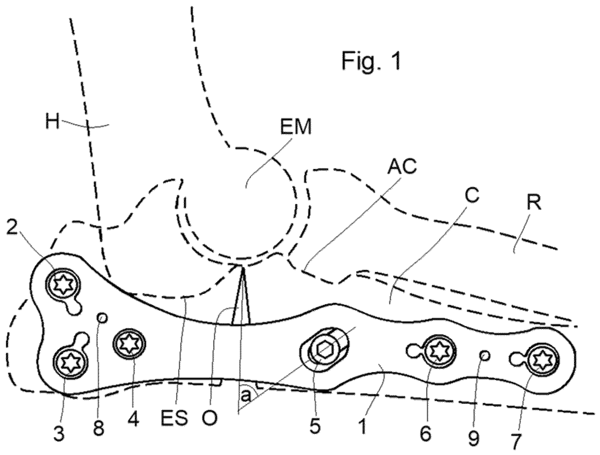

As can be seen in figure 1, the plate (1) is secured on top of the bone forming a connecting bridge between the two fragments into which the osteotomy (O) has divided the ulna (C), in order to stabilise them until their osseointegration is complete.

Three areas are distinguished on this plate (1): proximal (17), which, once secured to the bone, is located corresponding to the proximal fragment of the ulna; intermediate (18), which once secured is positioned above the osteotomy (O); and distal (19), which, once secured, is positioned along the ulna (C).

The proximal area (17) has at least three holes (11, 12, 13) for securing therein securing screws (2, 3, 4). The intermediate zone (18) has an elongate and concave shape on its side facing the medial humeral epicondyle (EM) and the ulnar coronoid process (AC) in order to avoid interference in this area. Lastly, the distal area (19) has an elongate configuration and along the length thereof there are at least three holes (14, 15, 16), in one of which a compression screw (5) is secured, while securing screws (6, 7) are engaged in the remaining holes. In a preferred embodiment, the hole (14) in which the compression screw (5) is placed is located in the distal part (19) of the piece (1), in the area closest to the intermediate area (18) covering the osteotomy (O).

In a preferred embodiment, the three holes (11, 12, 13) into which the securing screws (2, 3, 4) are engaged in the proximal area of the ulna have a triangular arrangement to ensure that they engage in this area despite the fact that it is subjected to greater stresses due to the action of the triceps thereon.

The securing screws (2, 3, 6, 7) are of the polyaxial type in order to allow a certain range of angulation with respect to the plate (1) when secured. In a preferred embodiment, the screws used are of the type described in the applicant's patent EP3738534B1.

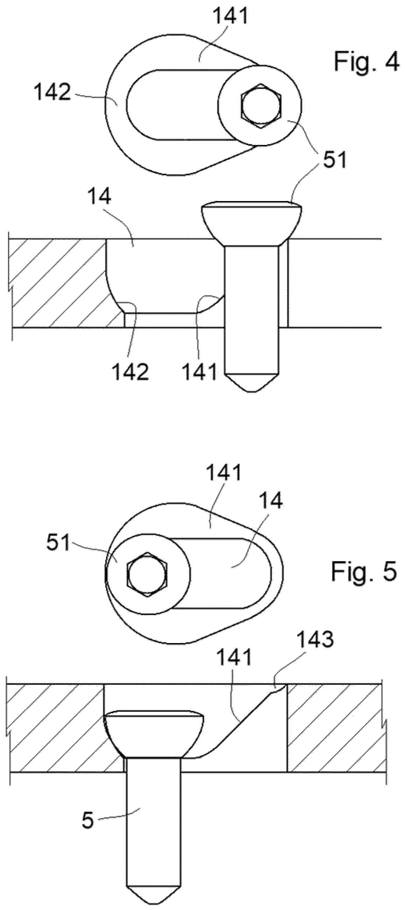

The compression screw (5) has a spherical or frustoconical head. The hole (14) in the plate (1), in which the compression screw is arranged, is shaped as an elongate slot that allows a slight longitudinal displacement of the screw intended to pass through it, with an inner ramp (141) running inwards from the distal to the proximal end of the plate (1), such that when the screw (5) is tightened, a compression effect is generated between the bone fragments of the distal and proximal part of the ulna, tending to close the wedge of the osteotomy (O) (see Figs. 4 and 5).

The hole (14), in which the compression screw (5) is arranged, forms an angle with the axis of the wedge osteotomy (O) between 40 and 70°, in order to avoid deformation of the bridge in the semilunate notch (ES) of the ulna by generating compression between the bone fragments of the distal and proximal part of the ulna, tending to close the wedge of the osteotomy (O). In a preferred embodiment, this hole is mounted forming an angle with the axis of the wedge osteotomy (O) between 45° and 55°.

The length of the elongate slot of the hole (14) in which the compression screw (5) is arranged has been calculated to be such that when compression is applied between the two fragments into which the distal and proximal part of the ulna (C) has been separated it allows the closure of a wedge formed between said fragments of an osteotomy (O) of up to 20°; the maximum distance the compression screw (5) can travel is 4 mm.

The ramp (141) on the longitudinal walls of the hole (14) in which the compression screw (5) is arranged has an angle of inclination between 30 and 60°. In a preferred embodiment, the angle of the ramp is approximately 45°.

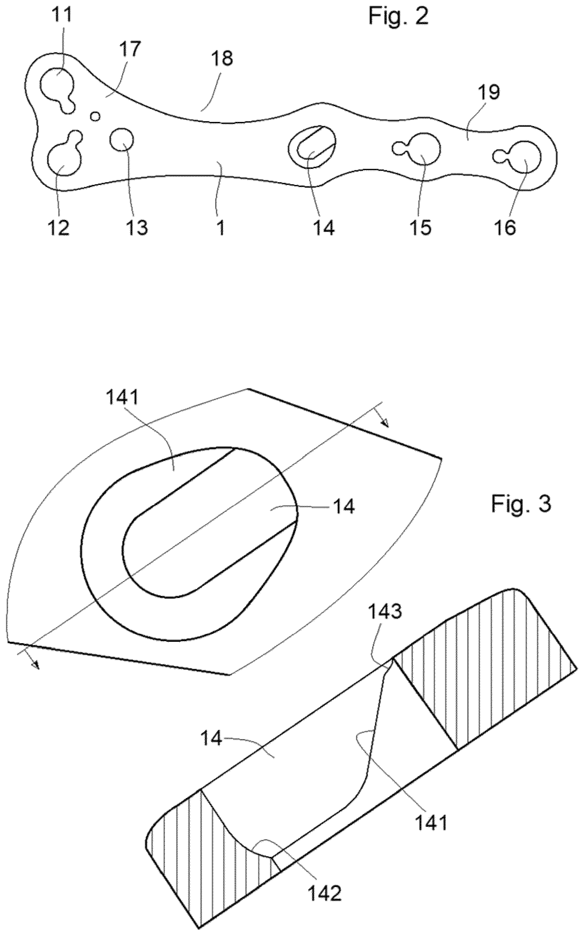

As shown in Fig. 3, the ramp (141) on the side walls of the hole (14) ends at the upper part of the piece (1) in a semilunate notch (143) in which the compression screw (5) is positioned before starting the compression of the osteotomy (O). While the wall of the hole (14) in the part ending in the deepest area, closest to the lower part of the piece (1), has a notch (142) with an arched shape in accordance with the head (51) of the compression screw (5).

As shown in Fig. 2, the plate (1) further comprises at least one hole (8) in the proximal area and at least one hole (9) in the distal area into which a clamping member of the plate (1) is initially inserted prior to securing via the securing screws.

To use a plate of this type, after having performed the wedge osteotomy (O) in the proximal area of the ulna (C), the plate is placed on this bone and, firstly, the three screws (2, 3, 4) are secured to the proximal part of the bone, which is considered secured, then the compression screw (5) is inserted into the hole (14) with an elongate slot and screwed into the second part of the bone in question. At the end of the screwing, when the threaded rod thereof is firmly secured inside the bone, the lower part of the head (51) thereof meets the ramp (141) on the inclined lateral edges of said hole (14), which causes a longitudinal displacement of said head (51) in the direction of the proximal part of the ulna, resulting in an equal displacement of the proximal part, in which this screw (5) is secured. This screwing and the corresponding displacement take place until the two parts where the wedge osteotomy (O) has separated the ulna are in place, attached or in contact with each other.

It is stated for appropriate purposes that the materials, shape, size and arrangement of the elements described may be modified, as long as this does not imply an alteration of the essential features of the invention that are claimed below:

## Claims

1. A plate for proximal ulnar closing wedge osteotomy for pet animals, which is a surgery to increase the radius of curvature of the semilunate notch (ES) of the ulna (C) by performing for this purpose an osteotomy (O) opening outwards but keeping an area in the semilunate notch (ES) of the ulna intact, wherein a plate (1) is secured on top of the bone forming a connecting bridge between the two fragments into which the osteotomy (O) has divided the ulna (C), in order to stabilise them until their osseointegration is complete, said plate (1) comprising:
- a proximal area (17), which, once secured to the bone, is located corresponding to the proximal fragment of the ulna, which is provided with at least three holes (11, 12, 13) for securing therein securing screws (2, 3, 4);
- an intermediate area (18), which once secured is located above the osteotomy (O), which has an elongate and concave shape on its side facing the medial humeral epicondyle (ME) and the ulnar coronoid process (AC); and
- a distal area (19), which, once secured, is positioned along the ulna (C), which has an elongate configuration and along the length thereof there are at least three holes (14, 15, 16), in one of which a compression screw (5) is secured, while securing screws (6, 7) are engaged in the remaining holes;
wherein the compression screw (5) has a spherical or frustoconical head, while the hole (14) in the plate (1) in which said compression screw (5) is arranged has an elongate slot with an inner ramp (141) starting from the upper face towards the lower face of the plate (1), from its distal to its proximal end, such that when the screw (5) is tightened, a compression effect is generated between the bone fragments of the distal and proximal part of the ulna, tending to close the wedge of the osteotomy (O);
wherein the securing screws (2, 3, 6, 7) are of the polyaxial type in order to allow a certain range of angulation with respect to the plate (1) when secured.

2. The plate according to claim 1, wherein the arrangement of the elongate slot of the hole (14) in which the compression screw (5) is arranged, forms an angle (a) with the axis of the wedge osteotomy (O) between 35 and 75°, in order to avoid deformation of the bridge in the semilunate notch (ES) of the ulna by generating compression between the bone fragments of the distal and proximal part of the ulna, tending to close the wedge of the osteotomy (O).

3. The plate according to any of the preceding claims, wherein the length of the elongate slot of the hole (14) in which the compression screw (5) is arranged is such that when compression is applied between the two bone fragments of the distal and proximal part of the ulna, it allows the closure of a wedge formed between the fragments into which it is divided in the osteotomy (O) of up to 15°.

4. The plate, according to any of the preceding claims, wherein the ramp (141) in the longitudinal walls of the hole (14) in which the compression screw (5) is arranged has an angle of inclination between 30 and 60°.

5. The plate, according to any of the preceding claims, wherein the ramp (141) on the side walls of the hole (14) ends at the upper part of the piece (1) in a semilunate notch (143) in which the compression screw (5) is positioned before starting the compression of the osteotomy (O).

6. The plate, according to any of the preceding claims, wherein the wall of the hole (14) in the part ending in the deepest area, closer to the lower part of the piece (1), has a notch (142) with an arched shape in accordance with the head (51) of the compression screw (5).

7. The plate, according to any of the preceding claims, wherein the hole (14) in which the compression screw (5) is placed is located in the distal part (19) of the piece (1), in the area closest to the intermediate area (18) covering the osteotomy (O).

8. The plate, according to any of the preceding claims, wherein the head (51) of the compression screw (5) has a hemispherical or frustoconical shape.

9. The plate, according to any of the preceding claims, further comprising at least one hole (8) in the proximal area and at least one hole (9) in the distal area into which a clamping member of the plate (1) is initially inserted prior to securing via the securing screws.
